# EUROPEAN PATENT APPLICATION

(11) **EP 4 173 483 A1**
(43) Date of publication of application: **03.05.2023**
(21) Application number: 21829034.4
(22) Date of filing: 25.06.2021
(51) Int. Cl.: A01N 1/02, B01L 3/00

(54) **CRYOPRESERVATION METHOD FOR ORGAN-ON-A-CHIP**

(30) Priority: 26.06.2020 KR 20200078256
(71) Applicant: Mepsgen Co., Ltd., Seoul 05836 (KR)
(72) Inventor: RHO, Hoon Suk, Seoul 06344 (KR)
(74) Representative: Callaghan, Dayle Anne
(86) International application number: PCT/KR2021/008025
(87) International publication number: WO 2021/261961

(57) **Abstract**

The present invention relates to a method for cryopreserving and thawing an organ-on-a-chip that has a three-dimensional tissue structure and function. Specifically, the present invention relates to a method for cryopreserving and thawing an organ-on-a-chip that comprises cells and hydrogel and has a microchannel structure, whereby the organ-on-a-chip has an excellent effect of being able to maintain the structure and function of the three-dimensional tissue before and after cryopreservation and thawing.

## Description

### Technical Field

The present invention relates to a method for cryopreserving and thawing an organ-on-a-chip that has a three-dimensional tissue structure and function. Specifically, the present invention relates to a method for cryopreserving and thawing an organ-on-a-chip that comprises cells and hydrogel and has a microchannel structure, whereby the organ-on-a-chip is able to maintain the structure and function of the three-dimensional tissue before and after cryopreservation and thawing, so that the end user who purchased the frozen organ-on-a-chip can easily use it after thawing.

### Background Art

An organ-on-a-chip is a technology that implements a desired function by culturing cell tissues constituting a specific organ in a small chip, and thus simulating the morphology and physiological property of that organ. An organ-on-a-chip can be used to study in detail the behavior of cell tissues of a specific organ and the mechanism of physicochemical reactions in microenvironment, and can be used as a model for drug toxicity and efficacy evaluation and the like in new drug development.

For cryopreservation and thawing of simple cells or an organ-on-a-chip having a two-dimensional structure, currently known methods can be used. In general, the conventional cell freezing is performed after dispersing the cells in a cryopreservation solution by enzymatic treatment of the cells to make them in a suspended state, and freezing of suspended cell tissues such as organoids or spheroids is also performed in the same manner. When dispersed in a suspended state in a cryopreservation solution, the cryopreservation solution is included in a sufficiently large amount compared to the amount of cells, so that uniform transfer in terms of heat transfer can be achieved, and the conventional cell freezing method can be used because there are relatively few technical considerations (Han, Sung-Hoon, et al. World journal of gastroenterology 23.6 (2017): 964; He, Andy, et al. Biopreservation and biobanking 18.3 (2020): 222-227; and Clinton, James, and Penney McWilliams-Koeppen. Current protocols in cell biology 82.1 (2019): e66).

However, unlike the suspended cell tissue, an organ-on-a-chip that implements the structure and function of a three-dimensional organ tissue (hereinafter, a "three-dimensional organ-on-a-chip") uses a microfluid-based designed cell tissue to simulate the structure and function of the cell tissue present in the organ. Therefore, it is difficult to apply the existing general cryopreservation method due to many technical considerations.

That is, in terms of heat transfer and diffusion transfer of a cryoprotectant, in the suspended cell tissue, heat and protectant transfer occurs as a radial one-dimensional phenomenon, whereas in a three-dimensional organ-on-a-chip, it occurs three-dimensionally along a complex channel shape. In addition, since the extracellular matrix (ECM) is used in the case of a three-dimensional organ-on-a-chip in which a three-dimensional cell culture with a shape similar to that of the body is performed, the network connection of the cells formed inside the tissue is broken due to the expansion and contraction occurring while the ECM undergoes freezing and thawing, so that the function and structure of the cell body cannot be maintained as it is. Therefore, unlike the existing freezing of cells and suspended cell tissues, various technical problems must be considered in a method for cryopreserving a three-dimensional organ-on-a-chip.

In the suspended cell tissue, the function preservation of the tissue is evaluated by the size of the tissue, the viability of the constituent cells, the protein expression of the constituent cells, and the like, whereas the function of the tissue formed in a three-dimensional organ-on-a-chip is additionally evaluated by the cell-to-cell contact and bonding force, the material permeability rate of a tissue, the polarization of a specific cell, and the like. The tissue function of only the three-dimensional organ-on-a-chip is a very important indicator for evaluating and predicting drug permeability rate, drug efficacy and toxicity in the human body, and maintaining the structure and function of the tissue during freezing and thawing of the three-dimensional organ-on-a-chip is important among others. However, a method for cryopreserving and thawing an organ-on-a-chip capable of maintaining the structure and function is not currently known.

Therefore, the present inventors developed a method capable of maintaining the structure and function of the organ tissue that it had before freezing even after cryopreservation and thawing of the three-dimensional organ tissue. Based on the above, the present inventors completed the present invention.

### Prior Art Document

### Patent Document

(Patent Document 0001) Korean Patent Publication No. 10-2017-0139048

### Non-Patent Document

(Non-Patent Document 0001) Han, Sung-Hoon, et al. "Long-term culture-induced phenotypic difference and efficient cryopreservation of small intestinal organoids by treatment timing of Rho kinase inhibitor." World journal of gastroenterology 23.6 (2017): 964
(Non-Patent Document 0002) He, Andy, et al. "Cryopreservation of viable human tissues: Renewable resource for viable tissue, cell lines, and organoid development." Biopreservation and biobanking 18.3 (2020): 222-227
(Non-Patent Document 0003) Clinton, James, and Penney McWilliams-Koeppen. "Initiation, expansion, and cryopreservation of human primary tissue-derived normal and diseased organoids in embedded three-dimensional culture." Current protocols in cell biology 82.1 (2019): e66

### Detailed Description of the Invention

### Technical Problem

An object of the present invention is to provide a method for cryopreserving an organ-on-a-chip that implements the structure and function of a specific organ or tissue, wherein the organ-on-a-chip undergoes the process of cryopreserving, and then the organ-on-a-chip is able to maintain the structure and function of the specific organ or tissue that it had before freezing even after freezing and thawing of the organ-on-a-chip, so that the end user can easily use the organ-on-a-chip after thawing in laboratories, research institutes, pharmaceutical companies, and the like.

In addition, another object of the present invention is to preserve the TEER (transepithelial electrical resistance) value, which is a representative index for evaluating the cell-to-cell bonding force, the material permeability rate of the tissue and the like in the tissue function of a three-dimensional organ-on-a-chip, even after freezing and thawing.

### Solution to Problem

The present invention provides a method for cryopreserving an organ-on-a-chip, comprising a step of preparing an organ-on-a-chip comprising an organ-on-a-chip tissue part comprising a second channel that comprises cells and hydrogel and has a microchannel structure, and an organ-on-a-chip barrier part comprising a first channel that comprises cells and has a microchannel structure, a step of perfusing a preservation solution containing a cryoprotectant through the microchannel included in the organ-on-a-chip barrier part, a step of refrigerated storage of the organ-on-a-chip, and a step of cooling and freezing the organ-on-a-chip.

The step of perfusing a preservation solution includes a step of flowing a preservation solution through the first channel for a certain period of time using a difference in hydrostatic pressure in order to uniformly distribute the preservative solution in the microchannel; and a step of replacing the preservative solution in a side channel using a pipette.

The step of refrigerated storage of the organ-on-a-chip includes a step of refrigerated storage for a certain period of time so that the preservative solution injected into the microchannel is uniformly diffused into the second channel, which is a three-dimensional hydrogel channel.

In addition, the present invention provides a method for thawing an organ-on-a-chip, comprising a step of thawing the organ-on-a-chip frozen according to the above method.

### Effects of the Invention

The cryopreservation and thawing method of the present invention allows the three-dimensional tissue or organ stored frozen to maintain the structure and function that it had before freezing even after thawing, so that the organ-on-a-chip can be easily used in laboratories, research institutes, pharmaceutical companies, and the like.

In particular, through the cryopreservation and thawing method of the present invention, the structure and function of a tissue barrier or a three-dimensional tissue barrier created by combining two-dimensional-three-dimensional tissues are maintained, and the cell viability and cell function of cells and tissue barrier cells are maintained at 90% or more, and the expression of proteins such as receptor proteins necessary to maintain the function and structure of the tissue is maintained.

In addition, the cryopreservation and thawing method of the present invention allows it to maintain the structure and function of the tissue barrier that it had before cryopreservation and to preserve the TEER value and permeability and the like, and thus to be capable of being usefully utilized to evaluate the efficacy and toxicity and the like of new drugs.

### Brief Description of Drawings

Figures 1 and 2 are a perspective view and a cross-sectional view of an organ-on-a-chip comprising a first channel and a second channel.
Figures 3 and 4 are a perspective view and a cross-sectional view of an organ-on-a-chip comprising a first channel, a second channel, and a side channel.
Figures 5 and 6 are a perspective view and a cross-sectional view of an organ-on-a-chip comprising a first channel, a second channel, and a scaffold.
Figures 7 and 8 are a perspective view and a cross-sectional view of an organ-on-a-chip comprising a first channel, a second channel, a side channel, and a scaffold.
Figure 9 shows the TEER value according to the use of dimethyl sulfoxide (DMSO), glycerol and ethylene glycol (EG) as a cryoprotectant.
Figures 10 and 11 show the TEER value according to DMSO concentration and refrigerated storage.
Figure 12 shows a comparison of hydrogel expansion and contraction before freezing and after thawing of an organ-on-a-chip according to the method for cryopreserving and thawing an organ-on-a-chip of the present invention.
Figures 13 to 15 show a comparison of protein and nucleus expression before freezing and after thawing according to the method for cryopreserving and thawing an organ-on-a-chip of the present invention.

### Best Mode for Carrying out the Invention

Hereinafter, the embodiments and examples of the present application will be described in detail with reference to the accompanying drawings so that those of ordinary skill in the art to which the present invention belongs can easily practice the present invention. However, the present application can be implemented in various forms and is not limited to the embodiments and examples described herein.

Throughout the specification of the present application, unless otherwise stated, when a certain part "comprises" a certain constituent element, it means that the certain part may further comprise other constituent elements rather than exclude other constituent elements.

The present invention provides a method for cryopreserving an organ-on-a-chip, comprising a step of preparing an organ-on-a-chip comprising an organ-on-a-chip tissue part comprising a second channel that comprises cells and hydrogel and has a microchannel structure, and an organ-on-a-chip barrier part comprising a first channel that comprises cells and has a microchannel structure, a step of perfusing a preservation solution containing a cryoprotectant through the microchannel included in the organ-on-a-chip barrier part, a step of refrigerated storage of the organ-on-a-chip, and a step of cooling and freezing the organ-on-a-chip.

As used herein, the term "organ-on-a-chip" refers to a chip manufactured to implement the desired structure or function by culturing one or more cells constituting a specific organ and tissue in a small chip, and thus simulating the morphology and physiological property of that organ and tissue.

As used herein, the term "cell" refers to a biological cell, including a plant cell, an animal cell (such as, a mammalian cell), a bacterial cell and a fungal cell and the like.

As used herein, the term "hydrogel" is a hydrophilic polymer crosslinked by cohesive force such as covalent bonds, hydrogen bonds, van der waals bonds, or physical bonds, and refers to a material having a three-dimensional polymer network structure that is capable of swelling since it contains a large amount of water in an aqueous solution therein.

As used herein, the term "microchannel" or "microchannel structure" refers to a channel of microscopic size through which fluid can flow, and means a structure comprising a channel having a dimension of millimeter, micrometer, or nanometer.

As used herein, the term "preservation solution" or "cryopreservation solution" refers to a liquid including cryoprotective agents, and vehicle solutions that deliver cryoprotective agents to tissues and cells, and the like, and means to minimize damage to tissues and cells that accompany the process of freezing and thawing.

As used herein, the term "cryoprotectant" refers to an additive for minimizing damage to cells due to phenomenon of crystallization of water during freezing, and in the present application, includes dimethyl sulfoxide (DMSO), glycerol, ethylene glycol, and the like, but is not limited thereto. Preferably, it is dimethyl sulfoxide (DMSO) or glycerol, and most preferably, it is DMSO.

The concentration of the cryoprotectant in the preservation solution may be preferably 3 v/v% or more, more preferably 5 v/v% or more, and most preferably 10 v/v%.

In one embodiment, the preservation solution may further comprise fetal bovine serum (FBS).

In one embodiment, the "organ-on-a-chip barrier part" may further comprise a "first channel" and a "side channel" that is located on a side of the second channel and has a microchannel structure.

In one embodiment, the "first channel" may comprise tissue barrier cells. The tissue barrier cells may include vascular endothelial cells; skin cells; cancer cells; secretory gland cells; muscle cells; and epithelial cells of bronchi, large intestine, small intestine, pancreas, or kidney.

As used herein, the term "tissue barrier cell" refers to a cell that plays a role in maintaining a specific structure of a tissue, and plays a role in protecting the tissue from external stimuli. In addition, it refers to a cell that plays a role in selectively permeating a substance using a strong binding force between tissue barrier cells or an extracellular matrix, and in maintaining homeostasis in the concentration of the substance in the tissue. The tissue barrier cells include epithelial tissue cells and vascular endothelial cells.

In another embodiment, the organ-on-a-chip may further comprise a scaffold between the first channel and the second channel. The scaffold and the second channel may be in contact with each other or have an interval of 10 µm or less.

As used herein, the term "scaffold" refers to an artificially created extracellular matrix (ECM) for control of tissue construction and cell function. Preferably, the scaffold may include a major protein of the extracellular matrix based on a structure of a porous membrane, but is not limited thereto.

In one embodiment, the height of the microchannel structure may be 10 µm to 3 mm. Preferably, the height of the microchannel structure may be 100 to 500 µm. More preferably, the height of the microchannel structure may be 200 to 300 µm.

In one embodiment, the microchannel structure may comprise an inlet and an outlet capable of inducing and controlling the perfusion of the liquid agent. The method capable of inducing and controlling the perfusion may include a method of using a difference in hydrostatic pressure between the inlet and the outlet, and a method of connecting an external pump to the inlet and the outlet, but is not limited thereto.

The step of perfusing a preservation solution includes a step of flowing a preservation solution through the first channel several times (for example, 2 to 3 times) for several minutes (for example, 1 to 5 minutes) using a difference in hydrostatic pressure in order to uniformly distribute the preservative solution in the microchannel; and a step of replacing the preservative solution in a side channel several times (for example, 2 to 3 times) using a pipette.

The step of refrigerated storage of the organ-on-a-chip includes a step of refrigerated storage for a certain period of time so that the preservative solution injected into the microchannel is uniformly diffused into the second channel, which is a three-dimensional hydrogel channel. The refrigerated storage time may be less than 30 minutes, preferably 10 to 20 minutes, and more preferably 15 minutes at 4 °C.

In one embodiment, the hydrogel may have a moisture content of 70% or more. Preferably, the hydrogel may have a moisture content of 80% or more.

In another embodiment, the hydrogel may include one or more selected from the group consisting of collagen, laminin, hyaluronic acid, mineral, fibrin, fibronectin, elastin, peptide, polyethylene glycol, and alginate. Preferably, the hydrogel may be collagen gel, fibrin gel, laminin gel, Matrigel, animal derived tumor basement membrane extract gel, tissue decellularized extracellular matrix gel, peptide gel, polyethylene glycol gel, or alginate gel, but is not limited thereto.

In another embodiment, the expansion and contraction of the hydrogel before and after freezing may not exceed 20%. Preferably, the expansion and contraction of the hydrogel before and after freezing may not exceed 10%.

In one embodiment, the second channel may comprise internal tissue cells. The internal tissue cells may be one or more selected from the group consisting of astrocytes, pericytes, nerve cells, neural stem cells, glial cells, cardiac myocytes, smooth muscle cells, intestinal epithelial cells, keratinocytes, skin fibroblasts, podocytes, and glomerular endothelial cells, but are not limited thereto.

As used herein, the term "internal tissue cell" refers to a cell that makes up the organs and tissues of the human body, and includes all cells that make up the human body tissue including, but not limited to, epithelial tissue, muscle tissue, nervous tissue, or connective tissue.

In one embodiment, the second channel may further comprise a cell culture solution or a cell suspension. With respect to the hydrogel, the cell culture solution or cell suspension may be included in a weight ratio of 0.1 to 1.

In one embodiment, the concentration of cells included in the second channel may be 10⁵ to 10⁷ cells/ml, and the number of cells included in the second channel may be 10³ to 10⁵.

In one embodiment, the preservation solution may be perfused at a flow rate of 1 to 200 µl/min for at least 30 seconds, and in another embodiment, the preservation solution may be perfused at room temperature. Preferably, the preservation solution may be perfused at a temperature of 4 to 10 °C.

In another embodiment, the preservation solution may include one or more selected from the group consisting of growth factors, drugs, soluble factors including proteins and nucleic acids, insoluble factors, and nanomaterials.

As used herein, the term "growth factor" refers to a substance that promotes cellular activities such as growth, division, recovery and differentiation of cells, including cytokines, hormones, nucleic acids, etc. secreted by cells. Growth factor is a type of soluble factor, and it means that it spreads in the liquid phase by cell secretion, such as VEGF, EGF, FGF, insulin, and growth hormone.

As used herein, the term "soluble factor" refers to a factor that can be absorbed through a receptor into a cell.

As used herein, the term "insoluble factor" refers to a factor that is not absorbed into a cell, such as extracellular matrix, extracellular matrix derived peptide, and glycosaminoglycan, and stimulates the cell from the outside.

As used herein, the term "nanomaterial" refers to a material such as nanoparticles, liposomes, graphene, etc. having a size of about 100 nm or less made through artificial synthesis.

In one embodiment, the cooling of the organ-on-a-chip may be performed at a rate of 0.5 to 5 °C/min until it reaches -80 °C. Preferably, the cooling of the organ-on-a-chip may be performed at a rate of 1 to 2 °C/min until it reaches -80 °C.

In another embodiment, after cooling the organ-on-a-chip to -80 °C, the method may further comprise a step of cooling the organ-on-a-chip, which reached -80 °C, to -196 °C using liquid nitrogen.

The present invention provides a method for freezing and thawing an organ-on-a-chip, comprising a step of preparing an organ-on-a-chip comprising an organ-on-a-chip tissue part comprising a second channel that comprises cells and hydrogel and has a microchannel structure, and an organ-on-a-chip barrier part comprising a first channel that comprises cells and has a microchannel structure, a step of perfusing a preservation solution containing a cryoprotectant through the microchannel included in the organ-on-a-chip barrier part, a step of refrigerated storage of the organ-on-a-chip, a step of cooling and freezing the organ-on-a-chip, and a step of thawing the frozen organ-on-a-chip.

As used herein, the term "thaw solution" is added during the thawing process, and refers to a solution for minimizing damage to cells or tissues that accompany thawing.

In one embodiment, the thawing of the organ-on-a-chip may be performed at 35 to 40 °C. Preferably, the thawing of the organ-on-a-chip may be performed at 37 °C.

In one embodiment, the thaw solution may be perfused at 35 to 40 °C at a flow rate of 8 to 32 µl/min or at a shear stress of 2 to 8 dyne/cm² for at least 30 seconds. In another embodiment, the thaw solution may include one or more selected from the group consisting of an animal cell medium; and growth factors, drugs, soluble factors including proteins and nucleic acids, insoluble factors, and nanomaterials.

The present invention provides the method for freezing and thawing an organ-on-a-chip, characterized in that the difference in permeability rate of the organ-on-a-chip before and after freezing and thawing is 20% or less.

The present invention provides the method for freezing and thawing an organ-on-a-chip, characterized in that the TEER value of the organ-on-a-chip before and after freezing and thawing is maintained at 80% or more, preferably 90% or more.

The present invention provides the method for freezing and thawing an organ-on-a-chip, characterized in that the expression of receptor proteins and cell tight j unction proteins of the organ-on-a-chip is maintained before and after freezing and thawing.

As used herein, the term "microfluidic device (millifluidic, microfluidic or nanofluidic device)" refers to a chip including a microchannel, etc. provided to allow a fluid to flow on a substrate made of various materials including plastic, glass, metal, or silicon including an organic polymer material.

As used herein, the term "culture solution" or "cell culture solution" refers to a solution having viable solution components and environments of cells. Components and concentrations are designed according to the properties of cells, and various commercially available cell culture solutions can be generally used as they are, or additional components can be added according to the properties of the target cells.

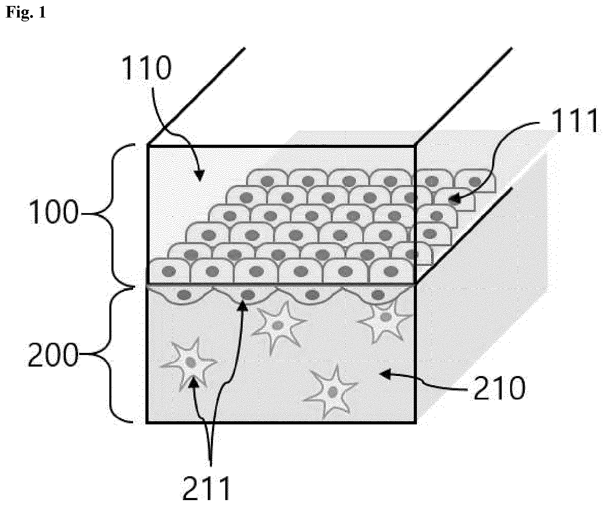

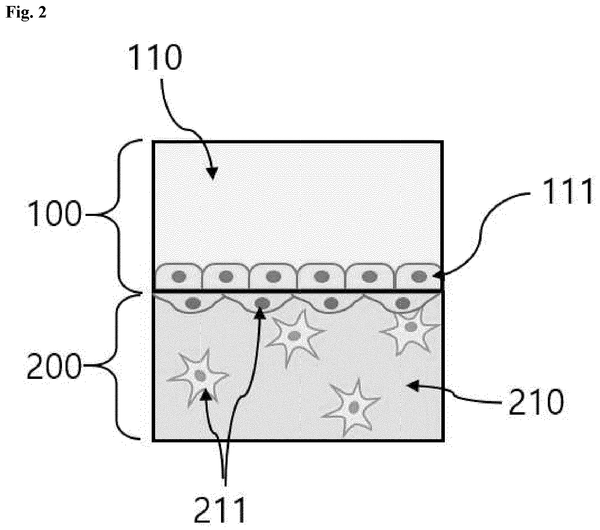

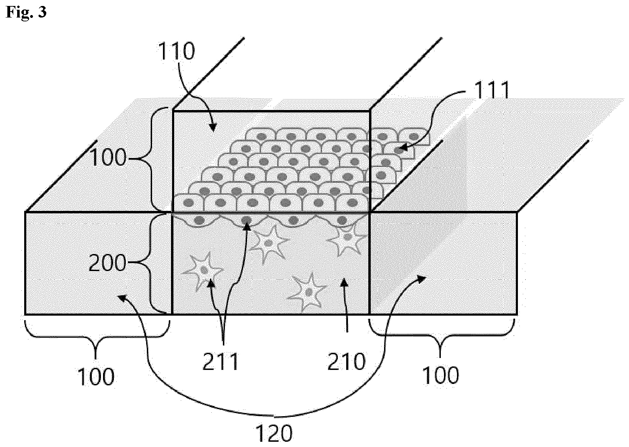

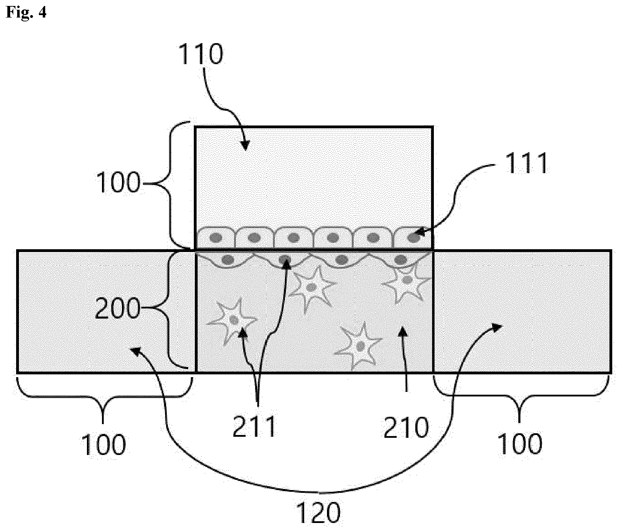

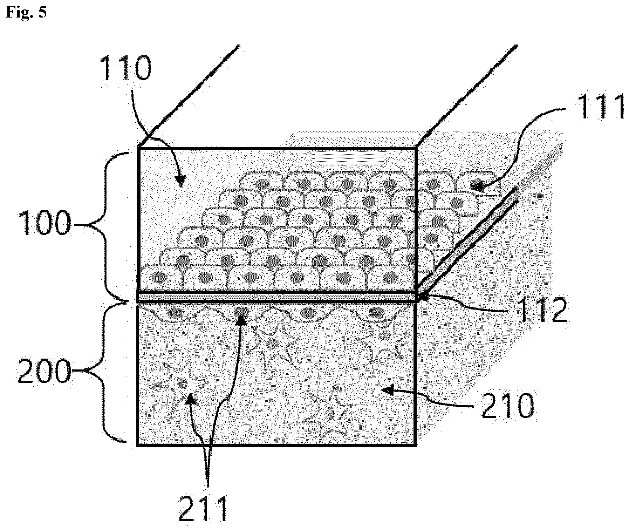

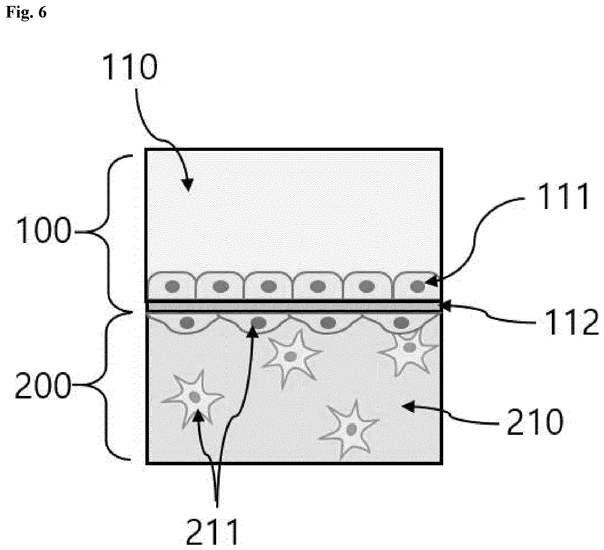

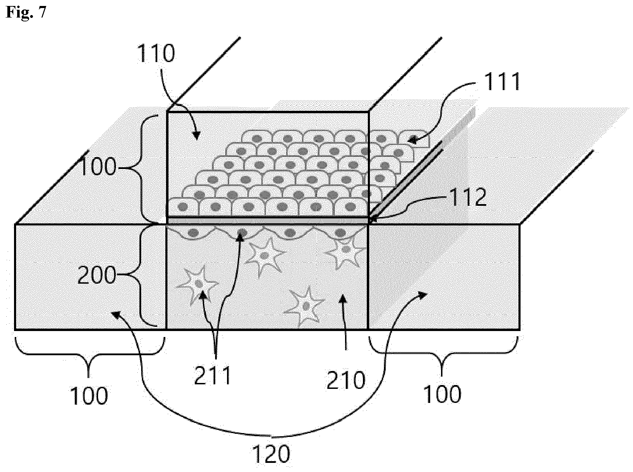

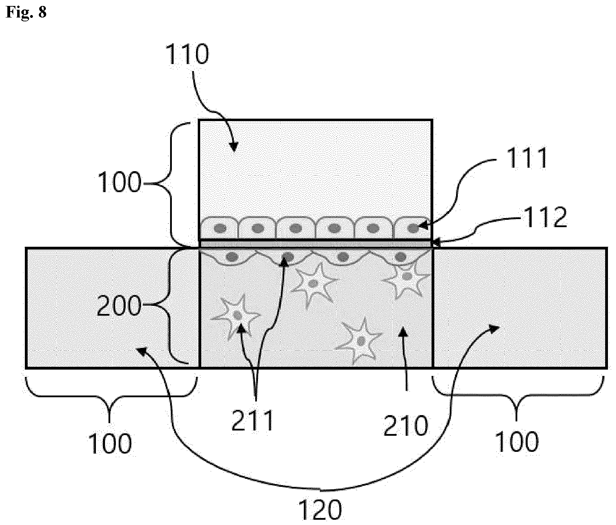

Hereinafter, the organ-on-a-chip of the present invention will be described in detail with reference to the drawings. The present invention may include an organ-on-a-chip embodied in the form of Figures 1 to 8, but is not limited thereto.

The organ-on-a-chip of the present invention comprises an organ-on-a-chip barrier part (100) and an organ-on-a-chip tissue part (200).

The organ-on-a-chip barrier part (100) comprises a first channel (110) that comprises tissue barrier cells (111) and has a microchannel structure. In addition, the organ-on-a-chip tissue part (200) comprises a second channel (210) that comprises internal tissue cells (211) and hydrogel and has a microchannel structure.

The organ-on-a-chip barrier part (100) may comprise a first channel (110) that comprises tissue barrier cells (111) and has a microchannel structure and a side channel (120) that is located on a side of the second channel (210) and has a microchannel structure.

The organ-on-a-chip of the present invention may comprise a scaffold (112) between the first channel and the second channel.

Hereinafter, the present invention is to be described in more detail through the following examples, but the following examples are for illustrative purposes only and are not intended to limit the scope of the present invention.

### [Example 1]

### Preparation of organ-on-a-chip

10 µl of fibronectin was injected into each of the first channel and the second channel of the organ-on-a-chip of the present invention, and cultured in an incubator for 1 hour. Cell adhesion promoters of the first channel and the second channel were removed, and 10⁷ cells/ml of a pericyte solution was injected into the second channel. After culturing for 3 hours in an incubator with the chip inverted, the culture solution in the second channel was removed. After injecting 10⁷ cells/ml of a mixture in which astrocytes and hydrogel were mixed in a 1:9 volume ratio into the second channel, they were cultured in an incubator for 30 minutes. The culture solution was injected into the side channel and the first channel, and the well reservoir was filled with the culture solution and cultured for one day, and then 10⁷ cells/ml of human brain microvascular endothelial cell (hBMEC) solution was injected into the first channel. After culturing for 1 hour in an incubator, the culture solution in the first channel was replaced and cultured for one day. The culture solution in the first channel and the side channel was replaced, and the side channel was plugged in, and then one side of the inlets of the first channel was connected to a syringe pump, and the other side was connected to a culture solution tank, and perfusion culture was performed at 4 dynes/cm² for 24 hours.

### [Example 2]

### Cryopreservation of organ-on-a-chip

All of the culture solution in the well reservoir was removed from the organ-on-a-chip of Example 1 above, and the first channel and the side channel were washed with the culture solution. The side channel was washed twice with the cryopreservation solution (50% culture solution, 40% FBS, 10% DMSO) at 4 to 10 °C. The culture solution in both inlets of the first channel was removed, and 30 µl of the cryopreservation solution was put into the inlet, and the cryopreservation solution was flowed by a difference in hydrostatic pressure (1 minute required). The preservation solution in the opposite inlet was removed, and 30 µl of the cryopreservation solution was additionally put into the inlet, and the cryopreservation solution was flowed by a difference in hydrostatic pressure (1 minute required). The chip was placed in a freezing container filled with isopropanol, and stored refrigerated for 15 minutes at 4 °C, and then stored in a deep freezer (-80 °C).

### [Example 3]

### Thawing of organ-on-a-chip

The organ-on-a-chip of Example 2 above was taken out of the deep freezer and thawed for 10 minutes by immersing it in a constant temperature water bath (37 °C) so that the bottom was in contact, and then the culture solution in the side channel was replaced twice with a culture solution at 37 °C. The preservation solution in both inlets of the first channel was removed, and 30 µl of the culture solution at 37 °C was put into the inlet, and the culture solution was flowed by a difference in hydrostatic pressure (1 minute required). The culture solution in the opposite inlet was removed, and 30 µl of the culture solution at 37 °C was additionally put into the inlet, and the culture solution was flowed by a difference in hydrostatic pressure (1 minute required). The well reservoir was filled with the culture solution at 37 °C and cultured for 24 hours or more.

### [Example 4]

### Confirmation of maintenance of tissue function according to cryoprotectant

In order to confirm the effect of maintaining tissue function according to the type of cryoprotectant, an experiment was performed according to the cryopreservation method of Example 2 above using each of dimethyl sulfoxide (DMSO), glycerol and ethylene glycol (EG) as a cryoprotectant, and the TEER (transepithelial electrical resistance) value was measured. The TEER value corresponds to a representative index for evaluating the cell-to-cell bonding force and the material permeability rate of the tissue and the like in the tissue function of the three-dimensional organ-on-a-chip. Since cells can continue to grow even after cryopreservation and thawing, it can be evaluated as an excellent cryoprotectant if the TEER value is maintained or increased when cultured for 24 hours after thawing.

The TEER value (ohm/cm²) was calculated as follows, and the TEER value before freezing was set to 1 and compared and evaluated with the TEER value after freezing (normalized).

### [cell cultured chip - chip (no cell)]

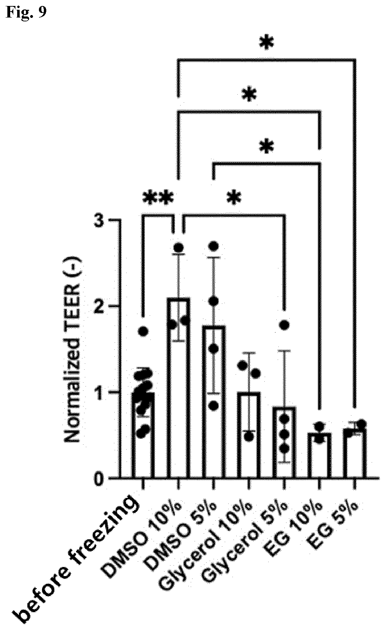

The experiment results are shown in Figure 9.

As shown in Figure 9, when EG was used as a cryoprotectant, it was confirmed that the TEER value was lower than the TEER value before freezing, indicating that EG was not appropriate as the cryoprotectant of the present invention.

In addition, it was confirmed that the TEER value of glycerol remained the same compared to the TEER value before freezing, and the TEER value of DMSO was increased, indicating glycerol and DMSO can be used as the cryoprotectant of the present invention, and DMSO is the most excellent cryoprotectant.

### [Example 5]

### Confirmation of maintenance of tissue function according to cryoprotectant (DMSO) and refrigerated storage

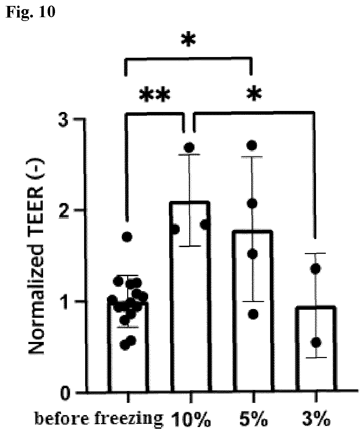

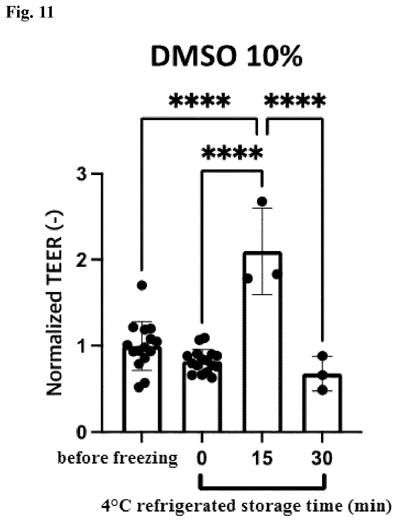

Using the cryopreservation method of Example 2 above, an experiment was performed to confirm the effect of maintaining the three-dimensional tissue function with various contents of dimethyl sulfoxide (DMSO). In addition, the effect of maintaining the three-dimensional tissue function according to 4 °C refrigerated storage was additionally confirmed. The TEER (transepithelial electrical resistance) value was measured in the same manner as in Example 4, and the results are shown in Figures 10 and 11.

As shown in Figure 10, it was confirmed that when the DMSO content was 3%, the TEER value remained the same as compared to the TEER value before freezing, and the TEER value was increased as the DMSO content was increased to 5% and 10%. Therefore, it can be seen that the DMSO content is preferably 3% or more, more preferably 5% or more, and most preferably 10%.

In addition, as shown in Figure 11, it was confirmed that the TEER value was increased when the refrigerated storage was performed for 15 minutes before cryopreservation compared to the case where the refrigerated storage was not performed. However, it was confirmed that the TEER value was rather reduced when the refrigerated storage was performed for 30 minutes before cryopreservation. Therefore, it can be seen that it is preferable to perform refrigerated storage for less than 30 minutes before cryopreservation, and it is more preferable to perform refrigerated storage for 15 minutes.

### [Example 6]

### Confirmation of expansion and contraction of hydrogel according to cryopreservation and thawing of organ-on-a-chip

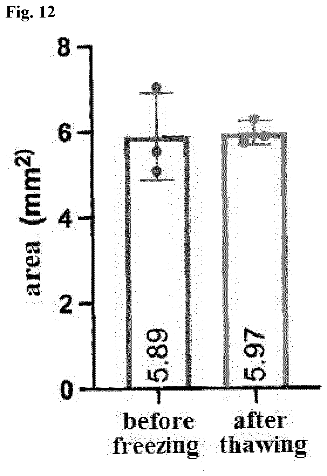

The expansion and contraction of hydrogel in the cryopreserved and thawed organ-on-a-chip were confirmed by using the method for cryopreserving and thawing an organ-on-a-chip of the present invention according to Examples 1 to 3 above. The results are shown in Figure 12.

As shown in Figure 12, the degree of expansion and contraction of the hydrogel before freezing and after thawing of the organ-on-a-chip according to the method for cryopreserving and thawing an organ-on-a-chip of the present invention was compared. As a result, it was confirmed that the hydrogel after thawing was expanded to a level of about 1.4% compared to that before freezing. Therefore, it can be seen that the organ-on-a-chip according to the cryopreservation and thawing method of the present invention maintains the structure of the tissue without deformation even after cryopreservation and thawing.

### [Example 7]

### Confirmation of protein expression according to cryopreservation and thawing of organ-on-a-chip

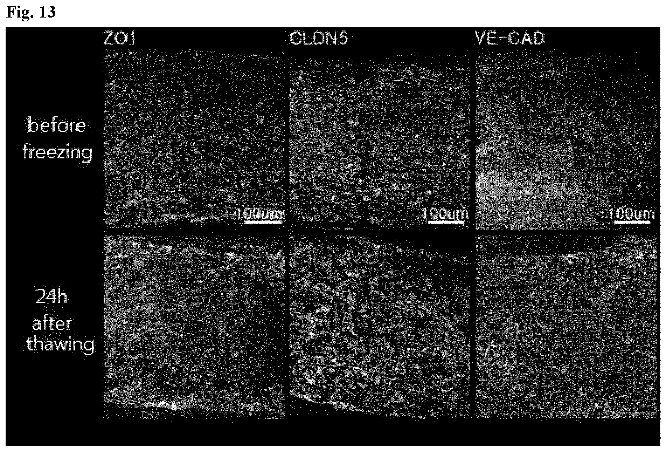

The protein expression and cell number of the cryopreserved and thawed organ-on-a-chip were confirmed by using the method for cryopreserving and thawing an organ-on-a-chip of the present invention according to Examples 1 to 3 above. The results are shown in Figures 13 to 15.

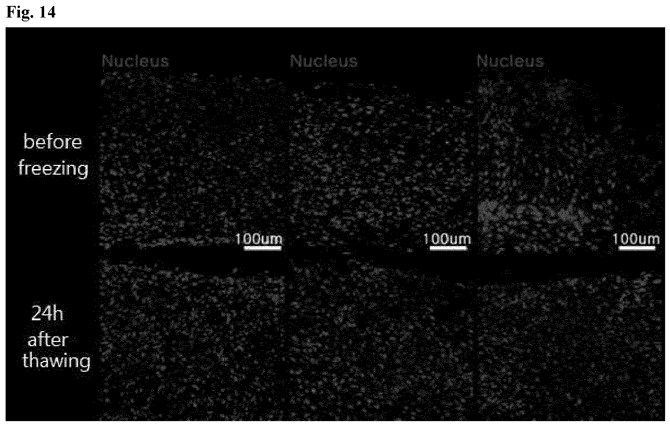

As shown in Figures 13 and 14, the protein expression before freezing and after thawing according to the method for cryopreserving and thawing an organ-on-a-chip of the present invention was compared. As a result, it was confirmed that even after thawing, the expression of cell junction proteins including ZO-1, VE-Cadherin and CLDN5, and the nucleus expression were maintained at a level similar to that before freezing.

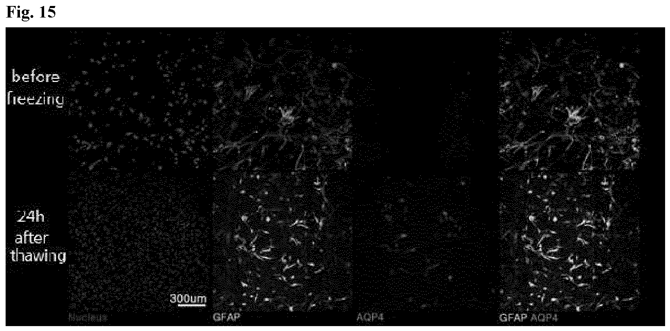

In addition, as shown in Figure 15, the protein expression before freezing and after thawing according to the method for cryopreserving and thawing an organ-on-a-chip of the present invention was compared. As a result, it was confirmed that even after thawing, the GFAP and AQP4 expression and nucleus expression of the three-dimensional astrocytes were maintained at a level similar to that before freezing.

Therefore, it can be seen that the organ-on-a-chip according to the cryopreservation and thawing method of the present invention maintains the number of cells and the structure and function of the tissue even after cryopreservation and thawing.

### Explanation of Code

100: organ-on-a-chip barrier part
110: first channel
111: tissue barrier cell
112: scaffold
120: side channel
200: organ-on-a-chip tissue part
210: second channel
211: internal tissue cell

## Claims

1. A method for cryopreserving an organ-on-a-chip, comprising
a step of preparing an organ-on-a-chip comprising an organ-on-a-chip tissue part comprising a second channel that comprises cells and hydrogel and has a microchannel structure, and an organ-on-a-chip barrier part comprising a first channel that comprises cells and has a microchannel structure;
a step of perfusing a preservation solution containing a cryoprotectant through the microchannel included in the organ-on-a-chip barrier part;
a step of refrigerated storage of the organ-on-a-chip; and
a step of cooling and freezing the organ-on-a-chip.

2. The method for cryopreserving an organ-on-a-chip according to claim 1, **characterized in that** the cryoprotectant is dimethyl sulfoxide (DMSO) or glycerol.

3. The method for cryopreserving an organ-on-a-chip according to claim 1, **characterized in that** the cryoprotectant is dimethyl sulfoxide (DMSO).

4. The method for cryopreserving an organ-on-a-chip according to claim 3, **characterized in that** the concentration of dimethyl sulfoxide (DMSO) in the preservation solution is 3 v/v% or more.

5. The method for cryopreserving an organ-on-a-chip according to claim 3, **characterized in that** the concentration of dimethyl sulfoxide (DMSO) in the preservation solution is 5 v/v% or more.

6. The method for cryopreserving an organ-on-a-chip according to claim 3, **characterized in that** the concentration of dimethyl sulfoxide (DMSO) in the preservation solution is 10 v/v%.

7. The method for cryopreserving an organ-on-a-chip according to claim 1, **characterized in that** the organ-on-a-chip is stored refrigerated for less than 30 minutes.

8. The method for cryopreserving an organ-on-a-chip according to claim 1, **characterized in that** the organ-on-a-chip is stored refrigerated for 10 to 20 minutes.

9. The method for cryopreserving an organ-on-a-chip according to claim 1, **characterized in that** the organ-on-a-chip is stored refrigerated at 4 °C for 15 minutes.

10. The method for cryopreserving an organ-on-a-chip according to claim 1, **characterized in that** the preservation solution further contains fetal bovine serum.

11. The method for cryopreserving an organ-on-a-chip according to claim 1, **characterized in that** the organ-on-a-chip barrier part further comprises a side channel that is located on a side of the second channel and has a microchannel structure.

12. The method for cryopreserving an organ-on-a-chip according to claim 1, **characterized in that** the first channel comprises tissue barrier cells.

13. The method for cryopreserving an organ-on-a-chip according to claim 12, **characterized in that** the tissue barrier cells include vascular endothelial cells; skin cells; cancer cells; secretory gland cells; muscle cells; and epithelial cells of bronchi, large intestine, small intestine, pancreas, or kidney.

14. The method for cryopreserving an organ-on-a-chip according to claim 1, **characterized in that** the organ-on-a-chip further comprises a scaffold between the first channel and the second channel.

15. The method for cryopreserving an organ-on-a-chip according to claim 14, **characterized in that** the scaffold and the second channel are in contact with each other or have an interval of 10 µm or less.

16. The method for cryopreserving an organ-on-a-chip according to claim 14, **characterized in that** the scaffold has a structure of a porous membrane.

17. The method for cryopreserving an organ-on-a-chip according to claim 1, **characterized in that** the hydrogel includes one or more selected from the group consisting of collagen gel, fibrin gel, laminin gel, animal derived tumor basement membrane extract gel, tissue decellularized extracellular matrix gel, peptide gel, polyethylene glycol gel, or alginate gel.

18. The method for cryopreserving an organ-on-a-chip according to claim 1, **characterized in that** the expansion and contraction of the hydrogel before and after freezing does not exceed 20%.

19. The method for cryopreserving an organ-on-a-chip according to claim 1, **characterized in that** the expansion and contraction of the hydrogel before and after freezing does not exceed 10%.

20. The method for cryopreserving an organ-on-a-chip according to claim 1, **characterized in that** the second channel comprises internal tissue cells.

21. The method for cryopreserving an organ-on-a-chip according to claim 20, **characterized in that** the internal tissue cells are one or more selected from the group consisting of astrocytes, pericytes, nerve cells, neural stem cells, glial cells, cardiac myocytes, smooth muscle cells, intestinal epithelial cells, keratinocytes, skin fibroblasts, podocytes, and glomerular endothelial cells.

22. A method for freezing and thawing an organ-on-a-chip, comprising
a step of preparing an organ-on-a-chip comprising an organ-on-a-chip tissue part comprising a second channel that comprises cells and hydrogel and has a microchannel structure, and an organ-on-a-chip barrier part comprising a first channel that comprises cells and has a microchannel structure;
a step of perfusing a preservation solution containing a cryoprotectant through the microchannel included in the organ-on-a-chip barrier part;
a step of refrigerated storage of the organ-on-a-chip;
a step of cooling and freezing the organ-on-a-chip; and
a step of thawing the frozen organ-on-a-chip.

23. The method for freezing and thawing an organ-on-a-chip according to claim 22, **characterized in that** the difference in permeability rate of the organ-on-a-chip before and after freezing and thawing is 20% or less.

24. The method for freezing and thawing an organ-on-a-chip according to claim 22, **characterized in that** the TEER value of the organ-on-a-chip before and after freezing and thawing is maintained at 80% or more.

25. The method for freezing and thawing an organ-on-a-chip according to claim 22, **characterized in that** the expression of receptor proteins and cell tight junction proteins of the organ-on-a-chip is maintained before and after freezing and thawing.
